# EUROPEAN PATENT APPLICATION

(11) **EP 4 703 465 A1**
(43) Date of publication of application: **04.03.2026**
(21) Application number: 23960662.7
(22) Date of filing: 21.12.2023
(51) Int. Cl.: C12N 5/079, C12N 5/0735, C12N 5/10, A61K 35/30, A61P 27/02, C12Q 1/02

(54) **PLURIPOTENT STEM CELL-DERIVED RPE CELL, FORMULATION, PHARMACEUTICAL COMPOSITION, REAGENT, METHOD, AND KIT**

(30) Priority: 08.12.2023 CN 202311679010
(71) Applicant: Help Regenerative Medicine Technology (Shenzhen) Co., Ltd., Shenzhen, Guangdong 518107 (CN)
(72) Inventor: WANG, Jiaxian, Nanjing, Jiangsu 211000 (CN); ZHANG, Yuehui, Nanjing, Jiangsu 211000 (CN)
(74) Representative: Epping - Hermann - Fischer
(86) International application number: PCT/CN2023/140578
(87) International publication number: WO 2025/118354

(57) **Abstract**

This invention, which pertains to the field of regenerative medicine and relates to cell therapy, provides a pluripotent stem cell-derived RPE cell, a preparation, a pharmaceutical composition, a reagent, a method, and a kit; said RPE cell is obtained through differentiation of iPSCs in an E6 medium supplemented with chemical small molecules serving as inhibitors or activators; said differentiation comprises the following stages: a neuroectodermal cell induction stage, an RPE progenitor cell induction stage, an RPE cell induction stage, and an RPE cell maturation stage. Manual removal of non-RPE-like heterogeneous cells is not required during said differentiation process, and pure RPE cells can be obtained through passaging culture.

## Description

### FIELD OF THE INVENTION

The present invention belongs to the field of regenerative medicine and relates to cell therapy, and in particular, to pluripotent stem cell-derived retinal pigment epithelium (RPE) cells, preparations, pharmaceutical compositions, reagents, methods, and kits.

### BACKGROUND OF THE INVENTION

The macula is a critical region of the retina, located at the posterior pole of the eye, and is primarily responsible for visual functions such as high-acuity vision and color perception. Pathological changes in the macular region often involve progressive loss of retinal pigment epithelium (RPE) cells, ultimately resulting in loss of visual function. Age-related macular degeneration (AMD) is a common, chronic, and progressive degenerative disease of the macula, with a global prevalence of approximately 8.7%. The pharmaceutical market of AMD represents a significant opportunity. Current mainstream treatment strategies for AMD fall into three major categories: laser-based intervention, surgical therapy, and pharmacological treatment.
(1) Neither laser-based intervention nor pharmacological treatment can cure neovascular AMD. As the number of injections increases, the efficacy of anti-VEGF drugs gradually diminishes, and in some patients, such treatments may even promote the progression of atrophic AMD. The annual cost of laser therapy ranges from RMB 4,000 to 60,000, while pharmacological treatment typically exceeds RMB 20,000 annually and requires long-term administration.
(2) Retinal pigment epithelium (RPE) cells form a monolayer of regularly arranged, tightly packed, polygonal pigmented cells located on the basal side of the retina, wherein the apical side of these cells is covered with numerous microvilli that surround the outer segments of photoreceptor cells (POS) for participating in phagocytosis. The basal side features extensive infoldings that facilitate substance exchange and connect to the choroid through Bruch's membrane, together forming the blood-retina barrier. In recent years, surgical therapy for RPE cells has become a hotspot for research. Multiple clinical trials of RPE cell transplantation have been conducted worldwide, with patients experiencing some degree of visual improvement one year after the operation. However, long-term use of immunosuppressants is required, and during extended follow-up, immune rejection has still been observed, with reduction in the number of RPE cells at the transplantation site and subsequent decline in visual acuity.

iPSC reprogramming technology: The emergence of human induced pluripotent stem cell (iPSC) technology has provided an ethically acceptable and unlimited source of cells for the treatment of macular degeneration. Although multiple clinical studies have confirmed the efficacy and safety of stem cell-derived RPE cells in treating macular degeneration, several limitations hinder clinical application. Autologous iPSC-derived RPE cells (hereinafter referred to as iPSC-RPE) require long differentiation periods (delaying treatment timing) and also incur high production costs and exhibit inconsistent quality. Meanwhile, allogeneic iPSC-RPE requires long-term use of immunosuppressants and fails to achieve permanent cell survival.

CRISPR/Cas9 Technology: With the continuous advancement of gene editing technologies, another major advantage of iPSC-RPE has become increasingly prominent, i.e., the ability to perform genome selection and gene editing (for immune evasion) at the iPSC stage. This approach addresses the challenge of immune rejection during clinical application of allogeneic iPSC-RPE, thereby enabling large-scale production of RPE cells and strict quality control prior to transplantation.

### Technical Problems

Technical problems and limitations in the differentiation of iPSCs into RPE cells: To date, most clinical studies and literature reports on RPE cell differentiation methods based on *in vivo* differentiation of RPE cells are spontaneous differentiation, which has the following problems:

This is labor-intensive and places high demands on technical personnel. Most differentiation methods still rely on manual selection of pigmented patches to achieve higher purity, making large-scale production of RPE cells extremely difficult. Moreover, if undifferentiated cells remain undetected in the final product, they pose a potential tumorigenic risk.

The differentiation period, yield, and quality standards vary significantly across different stem cell lines, resulting in poor reproducibility. For example, in Reference 1, the differentiation period is 60 days, and the markers used include: CD140b, CD56, CD104, CD184, and GD2, and the reported yield is 6,500-13,000 therapeutic doses of RPE cells from 1 million undifferentiated hiPSCs (Reference 1: Plaza Reyes, A. et al. Identification of cell surface markers and establishment of monolayer differentiation to retinal pigment epithelial cells. Nat Commun 11, 1609 (2020)); In Reference 2, the differentiation period is 42 days, with markers including LUM, FN1, MITF, and BEST, and the yield reaches 16 billion therapeutic RPE cells produced over 12 weeks using the automated robotic system (Reference 2: Regent, F., Morizur, L., Lesueur, L. et al. Automation of human pluripotent stem cell differentiation toward retinal pigment epithelial cells for large-scale productions. Sci Rep 9, 10646 (2019)).

At the same time, the differentiation methods described in the literature typically use animal-derived components to induce RPE differentiation. In recent years, with the growing understanding of *in vivo* and *in vitro* development of RPE cells, directed differentiation methods have gradually emerged.

### SUMMARY OF THE INVENTION

This invention provides a pluripotent stem cell-derived RPE cell, preparation, pharmaceutical composition, reagent, method, and kit. The differentiation process does not require manual removal of non-RPE-like heterogeneous cells and contains no animal-derived components. Pure iPSC-RPE cells can be obtained through passaging culture, thereby providing a safe and reliable RPE cell product for subsequent clinical trials.

To achieve the above technical objective, the present invention adopts the following technical solution: a pluripotent stem cell-derived RPE cell, wherein said RPE cell is obtained from pluripotent stem cell through the following differentiation steps:
A neuroectodermal cell induction stage, comprising inducing differentiation by inhibiting one or more signaling pathways selected from TGF-β/Nodal/Activin signaling pathway, BMP signaling pathway, and WNT signaling pathway;
An RPE progenitor cell induction stage, comprising inducing differentiation by activating Nicotinamide metabolic signaling pathway;
An RPE cell induction stage, comprising inducing differentiation by one or more of activating TGF-β/Nodal/Activin signaling pathway, inhibiting FGF signaling pathway, and activating WNT signaling pathway; and
An RPE cell maturation stage, comprising inducing differentiation by activating one or more signaling pathway selected from Nicotinamide metabolic signaling pathway and WNT signaling pathway.

As an improved technical solution of the present invention, said pluripotent stem cell is differentiated from ESC, iPSC, or universal iPSC^{HLA-KO};
Said universal iPSC^{HLA-KO} is an iPSC with immune-privileged properties obtained by gene editing to specifically reduce HLA expression.

As an improved technical solution of the present invention, said ESC is an ESC that has been passaged to 4-7 generations, and the split ratio for each passaging is 1:(3-12); the cultured ESC is digested into single cells and cultured in iPSC medium for one day, and then differentiated into RPE cells;
Said iPSC or said universal iPSC^{HLA-KO} is an iPSC that has been passaged to 4-7 generations, and the split ratio for each passaging is 1:(3-12); the cultured iPSC is digested into single cells and cultured in an iPSC medium for one day, and then differentiated into RPE cells.

As an improved technical solution of the present invention, said iPSC medium comprises E8, Stem Flex, or mTeSR1 as a basal medium, and is supplemented with 10 µM Y27632 as an additive.

As an improved technical solution of the present invention, said RPE cell is obtained through differentiation of iPSCs in an E6 medium supplemented with small chemical molecules serving as inhibitors or activators, said differentiation comprises the following stages: a neuroectodermal cell induction stage, an RPE progenitor cell induction stage, an RPE cell induction stage, and an RPE cell maturation stage.

As an improved technical solution of the present invention, said neuroectodermal cell induction stage comprises:
Inhibiting the TGF-β/Nodal/Activin signaling pathway by adding 1-50 µM of small molecule SB431542;
Inhibiting the BMP signaling pathway by adding 10-100 nM of small molecule LDN193183;
Inhibiting the WNT signaling pathway by adding 10-100 µM of small molecule IWR-1 or IWR-2.
As an improved technical solution of the present invention, said RPE progenitor cell induction stage comprises activating the Nicotinamide metabolic signaling pathway by adding 1-50 mM of Nicotinamide.

As an improved technical solution of the present invention, said RPE cell induction stage comprises:
Activating the TGF-β/Nodal/Activin signaling pathway by adding 10-200 ng/mL of Activin A;
Inhibiting the FGF signaling pathway by adding 1-10 µM of small molecule SU5402;
Activating the WNT signaling pathway by adding 1-10 µM of small molecule CHIR99021.

As an improved technical solution of the present invention, said RPE cell maturation stage comprises:
Activating the Nicotinamide metabolic signaling pathway by adding 1-50 mM of Nicotinamide;
Activating the WNT signaling pathway by adding 1-10 µM of small molecule CHIR99021.

As an improved technical solution of the present invention, the passaging split ratio of said RPE cell induction stage is 1:(3-20). The passaging ratio of said RPE cell maturation stage is 1:(3-20).

Another objective of this invention is to provide an RPE cell preparation, comprising said pluripotent stem cell-derived RPE cell of this invention.

Another objective of this invention is to provide a pharmaceutical composition for use in ophthalmology, comprising said pluripotent stem cell-derived RPE cell of this invention as an active ingredient.

Another objective of this invention is to provide a reagent for evaluating the toxicity or efficacy of a test substance, comprising said pluripotent stem cell-derived RPE cell of this invention.

Another objective of this invention is to provide a method for evaluating the toxicity or efficacy of a test substance, comprising contacting said pluripotent stem cell-derived RPE cell of this invention with said substance and determining the effect of said substance on said cell.
A further objective of this invention is to provide a kit, comprising a medium combination;
Said medium combination comprises: a first medium, a second medium, a third medium, and a fourth medium;
Said first medium comprises a basal medium and additive A, wherein said additive A is one or more selected from SB431542, LDN193183, IWR-1, and IWR-2 in any molar ratio; wherein SB431542 is added at 1-50 µM, LDN193183 at 10-100 nM, IWR-1 at 10-100 µM, and IWR-2 at 10-100 µM; Said second medium comprises a basal medium and additive B, wherein said additive B is Nicotinamide, which is added at 1-50 mM;
Said third medium comprises a basal medium and additive C, wherein additive C is one or more selected from Activin A, SU5402, and CHIR99021 in any molar ratio; wherein Activin A is added at 10-200 ng/mL, SU5402 at 1-10 µM, and CHIR99021 at 1-10 µM;
Said fourth medium comprises a basal medium and additive D, wherein additive D is one or both of Nicotinamide and CHIR99021 in any molar ratio; wherein Nicotinamide is added at 1-50 mM and CHIR99021 at 1-10 µM.

As an improved technical solution of the present invention, said basal medium is E6 medium.

A further objective of this invention is to provide a method of obtaining RPE cells using said kit of this invention, comprising the following steps:
Step 1: culturing said pluripotent stem cells in said first medium for 2 days to obtain neuroectodermal cells;
Step 2: culturing said neuroectodermal cells in said second medium for 4 days to obtain RPE progenitor cells;
Step 3: culturing said RPE progenitor cells in said third medium for 4 days to obtain RPE cells;
Step 4: culturing said RPE cells in said fourth medium for 20 days to obtain mature RPE cells.

### Advantages

In the prior art, the induction of differentiation of ESCs/iPSCs into RPE cells is often associated with unclear cellular states, low differentiation efficiency, and prolonged differentiation cycles (for example, Chinese Patent CN110573610A, titled "Method for Preparing Retinal Pigment Epithelial Cells," requires a differentiation period of 43 days). In contrast, the present invention integrates the differentiation process with the *in vivo* differentiation signaling pathways of RPE cell, which enables clear identification of cellular states during differentiation, significantly improves differentiation efficiency, and shortens the differentiation period to only 30 days. Moreover, the resulting RPE cells exhibit high purity and high yield.

The present invention clearly defines the signaling pathway activators or inhibitors to be added during the differentiation process, thereby enabling a standardized and efficiently reproducible differentiation method.

The differentiation process of the present invention does not involve any animal-derived components, eliminating the risk of exogenous viral contamination and improving safety for future clinical applications.

The differentiation process of the present invention is applicable to both iPSCs and immune-privileged universal iPSC^{HLA-KO} lines, with both exhibiting comparable efficiency in differentiating into RPE cells and yielding RPE cells with similar functional characteristics. Based on extensive experimental research, this invention confirms the broad applicability of said differentiation method for differentiating iPSCs toward RPE cells.

This invention integrates cutting-edge induced pluripotent stem cell (iPSC) technology with gene editing techniques to reduce immunogenicity at the iPSC stage. Moreover, the differentiation strategy integrates the *in vivo* differentiation stages of RPE cells and associated signaling pathways, thereby minimizing immune rejection during RPE cell transplantation.

In summary, the RPE cells obtained in this invention are highly safe, stable, donor-independent, and immunogenically unrestricted universal iPSC^{HLA-KO}-RPE cells, generated through directed differentiation induced by animal-component-free small molecules. These cells hold promises for the treatment and clinical translation of retinal degenerative diseases while addressing safety concerns, such as immune rejection, associated with allogeneic subretinal RPE cell transplantation in existing technologies.

### BRIEF DESCRIPTION OF THE FIGURES

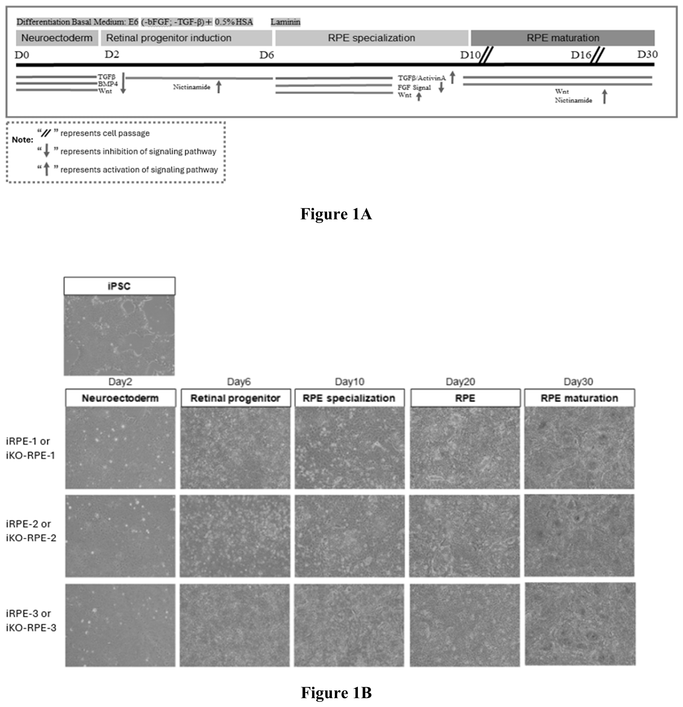

**Figure 1A** shows a flowchart illustrating the small molecule-induced differentiation process.
**Figure 1B** shows bright-field images of typical cellular morphology at various time points during the differentiation of iPSC-RPE or iPSC^{HLA-KO}-RPE cells.

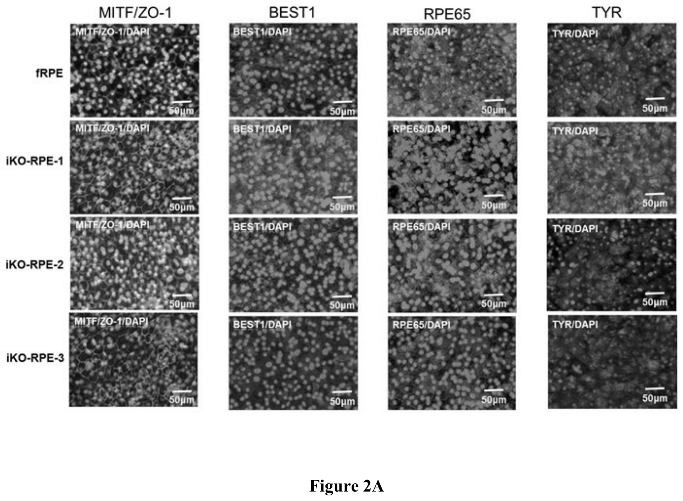

**Figure 2A** shows immunofluorescence analysis of specific protein expressions in differentiated cells iKO-RPE-1, iKO-RPE-2, iKO-RPE-3, and the positive control fRPE (fetal RPE). In the figure, iKO-RPE refers to RPE cells differentiated from iPSC^{HLA-KO} lines (abbreviated as iPSC^{HLA-KO-}RPE), and iRPE refers to RPE cells differentiated from iPSCs (abbreviated as iPSC-RPE).

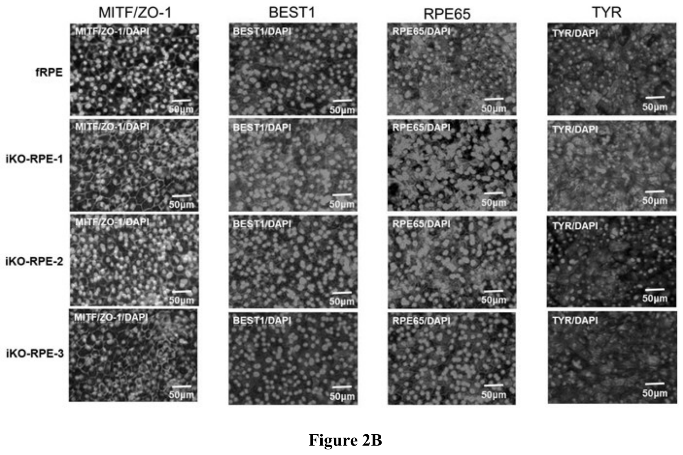

**Figure 2B** shows immunofluorescence analysis of specific protein expression in differentiated cells iRPE-1, iRPE-2, and iRPE-3. Cells iKO-RPE-1, iKO-RPE-2, and iKO-RPE-3 are iPSC^{HLA-KO}-RPE cells obtained using three different experimental methods as described in Example 1; iRPE-1, iRPE-2, and iRPE-3 are iPSC-RPE cells obtained using three different experimental methods as described in Example 2. In the figure, iKO-RPE cells refer to RPE cells differentiated from iPSC^{HLA-KO}-RPE lines (abbreviated as iPSC^{HLAKO-KO}-RPE), and iRPE cells refer to RPE cells differentiated from iPSCs (abbreviated as iPSC-RPE).

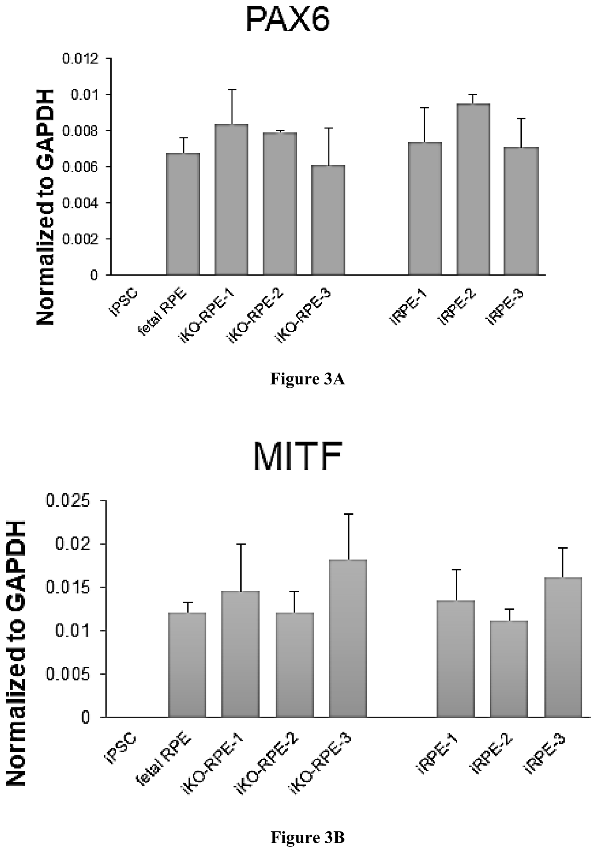

**Figure 3A** shows the expression of PAX6 in iPSCs, fRPE, iKO-RPE-1, iKO-RPE-2, iKO-RPE-3, iRPE-1, iRPE-2, and iRPE-3. In the figure, iKO-RPE refers to RPE cells differentiated from iPSC^{HLA-KO} lines (iPSC^{HLA-KO}-RPE), and iRPE refers to RPE cells differentiated from iPSCs (iPSC-RPE).
**Figure 3B** shows the expression of MITF in iPSCs, fRPE, iKO-RPE-1, iKO-RPE-2, iKO-RPE-3, iRPE-1, iRPE-2, and iRPE-3. In the figure, iKO-RPE refers to RPE cells differentiated from iPSC^{HLA-KO} lines (iPSC^{HLA-KO}-RPE), and iRPE refers to RPE cells differentiated from iPSCs (iPSC-RPE).

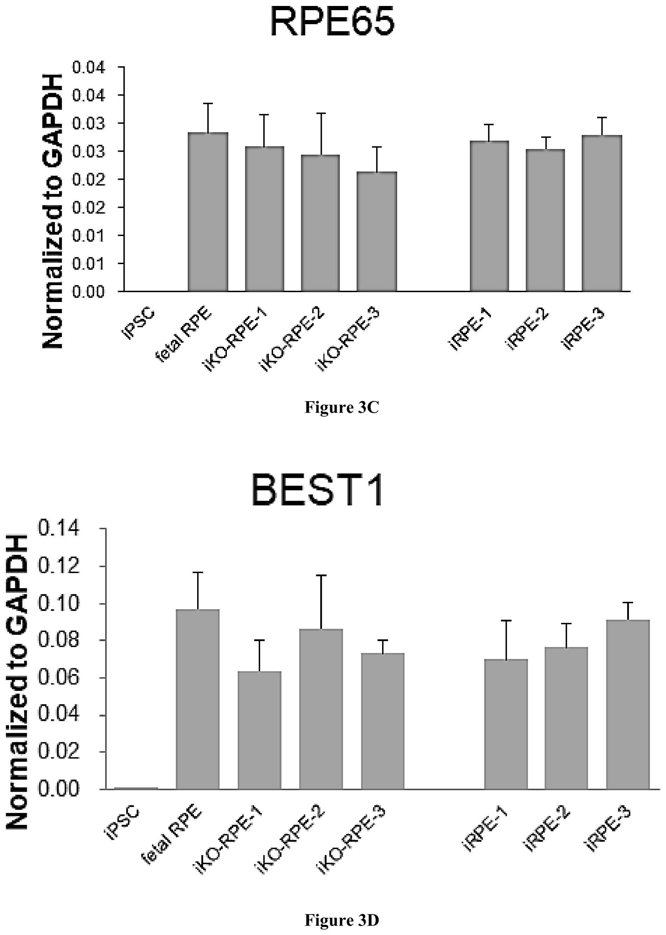

**Figure 3C** shows the expression of RPE65 in iPSCs, fRPE, iKO-RPE-1, iKO-RPE-2, iKO-RPE-3, iRPE-1, iRPE-2, and iRPE-3. In the figure, iKO-RPE refers to RPE cells differentiated from iPSC^{HLA-KO} lines (iPSC^{HLA-KO}-RPE), and iRPE refers to RPE cells differentiated from iPSCs (iPSC-RPE).
**Figure 3D** shows the expression of BEST1 in iPSCs, fRPE, iKO-RPE-1, iKO-RPE-2, iKO-RPE-3, iRPE-1, iRPE-2, and iRPE-3. In FIG. 3, "Normalized to GAPDH" refers to the expression level relative to GAPDH.

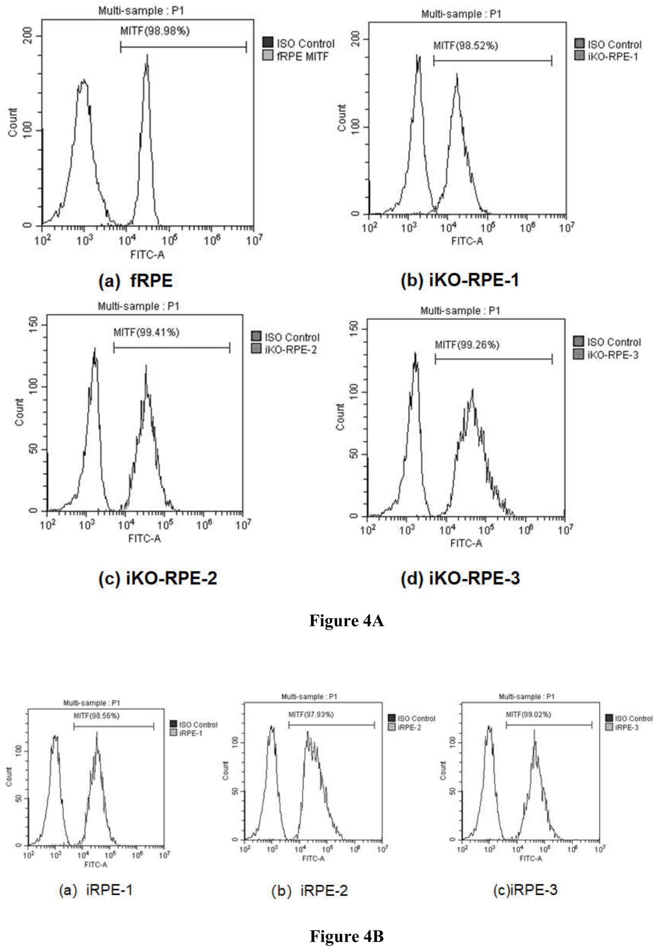

**Figure 4A** shows flow cytometry analysis of MITF protein expression in fRPE, iKO-RPE-1, iKO-RPE-2, and iKO-RPE-3 cells: A shows MITF expression in fRPE cells (98.98%); B shows MITF expression in iKO-RPE-1 cells (98.52%); C shows MITF expression in iKO-RPE-2 cells (99.41%); D shows MITF expression in iKO-RPE-3 cells (99.26%). In the figure, iKO-RPE refers to RPE cells differentiated from iPSC^{HLA-KO} lines (iPSC^{HLA-KO}-RPE), and iRPE refers to RPE cells differentiated from iPSCs (iPSC-RPE).
**Figure 4B** shows flow cytometry analysis of MITF protein expression in iPSC-RPE cells: A shows MITF expression in iRPE-1 cells (98.56%); B shows MITF expression in iRPE-2 cells (97.93%); C shows MITF expression in iRPE-3 cells (99.02%). iRPE-1, iRPE-2, and iRPE-3 were obtained using three different experimental methods described in Example 2. In the figure, iKO-RPE refers to RPE cells differentiated from iPSC^{HLA-KO} lines (iPSC^{HLA-KO}-RPE), and iRPE refers to RPE cells differentiated from iPSCs (iPSC-RPE).

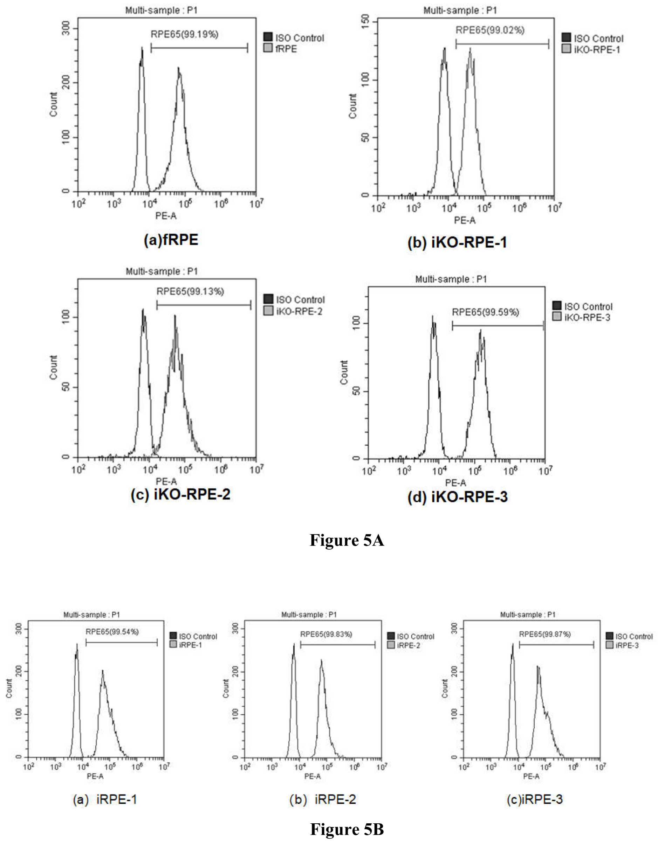

**Figure 5A** shows flow cytometry analysis of RPE65 protein expression in iPSC^{HLA-KO}-RPE cells and positive control fRPE cells: A shows RPE65 expression in fRPE cells (99.19%); B shows RPE65 expression in iKO-RPE-1 cells (99.02%); C shows RPE65 expression in iKO-RPE-2 cells (99.13%); D shows RPE65 expression in iKO-RPE-3 cells (99.59%). iKO-RPE-1, iKO-RPE-2, and iKO-RPE-3 were obtained using three different experimental methods described in Example 1. In the figure, iKO-RPE refers to RPE cells differentiated from iPSC^{HLA-KO} lines (iPSC^{HLA-KO}-RPE), and iRPE refers to RPE cells differentiated from iPSCs (iPSC-RPE).
**Figure 5B** shows flow cytometry analysis of RPE65 protein expression in iPSC-RPE cells: A shows RPE65 expression in iRPE-1 cells (99.54%); B shows RPE65 expression in iRPE-2 cells (99.83%); C shows RPE65 expression in iRPE-3 cells (99.87%). iRPE-1, iRPE-2, and iRPE-3 were obtained using three different experimental methods described in Example 2. In the figure, iKO-RPE refers to RPE cells differentiated from iPSC^{HLA-KO} lines (iPSC^{HLA-KO} -RPE), and iRPE refers to RPE cells differentiated from iPSCs (iPSC-RPE).

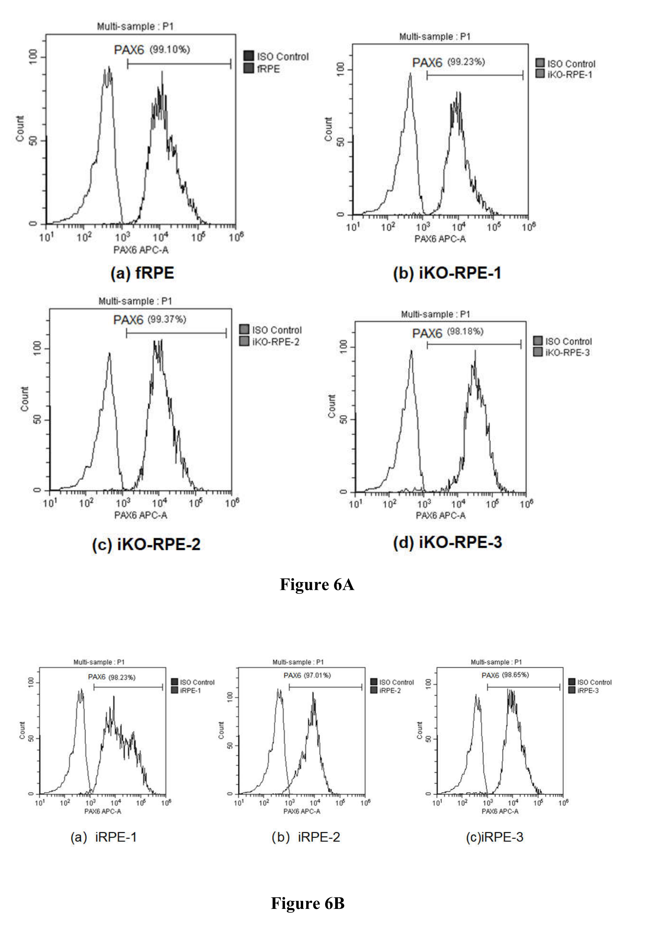

**Figure 6A** shows flow cytometry analysis of PAX6 protein expression in iPSC^{HLA-KO}-RPE cells and positive control fRPE cells: A shows PAX6 expression in fRPE cells (99.10%); B shows PAX6 expression in iKO-RPE-1 cells (99.23%); C shows PAX6 expression in iKO-RPE-2 cells (99.37%); D shows PAX6 expression in iKO-RPE-3 cells (98.18%). iKO-RPE-1, iKO-RPE-2, and iKO-RPE-3 were obtained using three different experimental methods described in Example 1. In the figure, iKO-RPE refers to RPE cells differentiated from iPSC^{HLA-KO} lines (iPSC^{HLA-KO}-RPE), and iRPE refers to RPE cells differentiated from iPSCs (iPSC-RPE).
**Figure 6B** shows flow cytometry analysis of PAX6 protein expression in iPSC-RPE cells: A shows PAX6 expression in iRPE-1 cells (98.23%); B shows PAX6 expression in iRPE-2 cells (97.01%); C shows PAX6 expression in iRPE-3 cells (98.65%). iRPE-1, iRPE-2, and iRPE-3 were obtained using three different experimental methods described in Example 2. In the figure, iKO-RPE refers to RPE cells differentiated from iPSC^{HLA-KO} lines (iPSC^{HLA-KO}-RPE), and iRPE refers to RPE cells differentiated from iPSCs (iPSC-RPE).

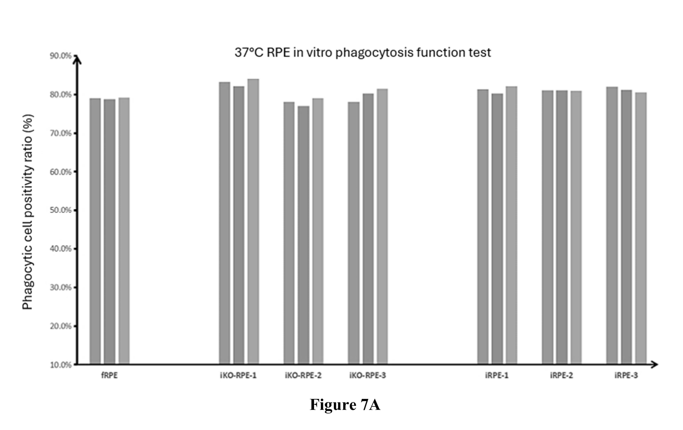

**Figure 7A** shows phagocytosis assay results at 37°C for fRPE, iRPE (iRPE-1, iRPE-2, and iRPE-3), and iKO-RPE (iKO-RPE-1, iKO-RPE-2, and iKO-RPE-3) cells. In the figure, iKO-RPE refers to RPE cells differentiated from iPSC^{HLA-KO} lines (iPSC^{HLA-KO}-RPE), and iRPE refers to RPE cells differentiated from iPSCs (iPSC-RPE).

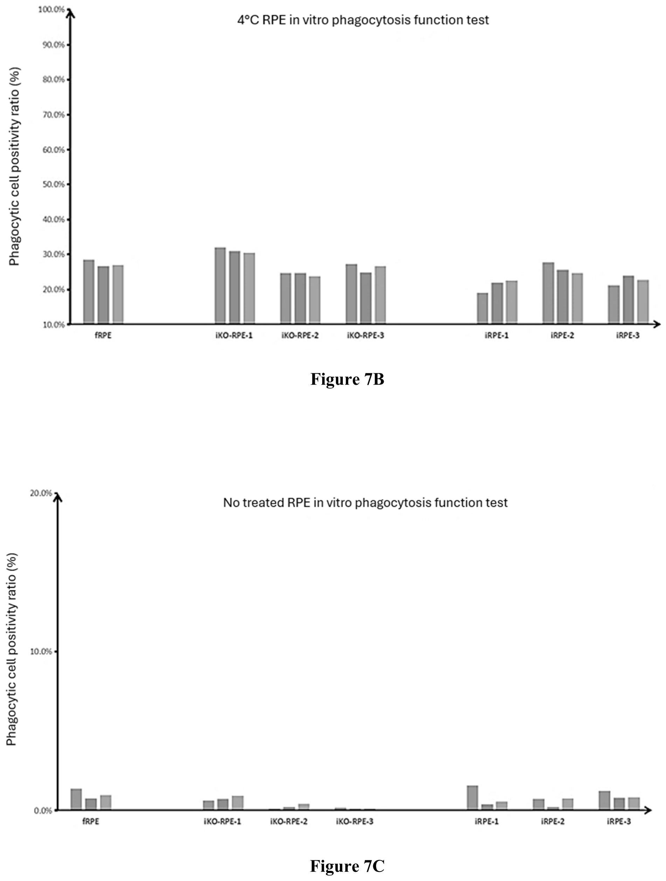

**Figure 7B** shows phagocytosis assay results at 4°C for fRPE, iRPE (iRPE-1, iRPE-2, and iRPE-3), and iKO-RPE (iKO-RPE-1, iKO-RPE-2, and iKO-RPE-3) cells. In the figure, iKO-RPE refers to RPE cells differentiated from iPSC^{HLA-KO} lines (iPSC^{HLA-KO} -RPE), and iRPE refers to RPE cells differentiated from iPSCs (iPSC-RPE).
**Figure 7C** shows the negative control group for fRPE, iRPE (iRPE-1, iRPE-2, and iRPE-3), and iKO-RPE (iKO-RPE-1, iKO-RPE-2, and iKO-RPE-3) cells. In the figure, iKO-RPE refers to RPE cells differentiated from iPSC^{HLA-KO} lines (iPSC^{HLA-KO}-RPE), and iRPE refers to RPE cells differentiated from iPSCs (iPSC-RPE).

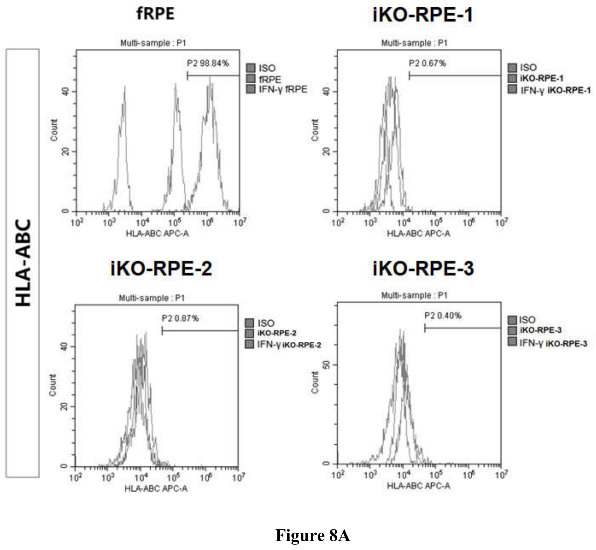

**Figure 8A** shows the assay results for class I immune-privileged characteristics of the HLA gene cluster encoding HLA-A, HLA-B, and HLA-C (HLA-ABC).

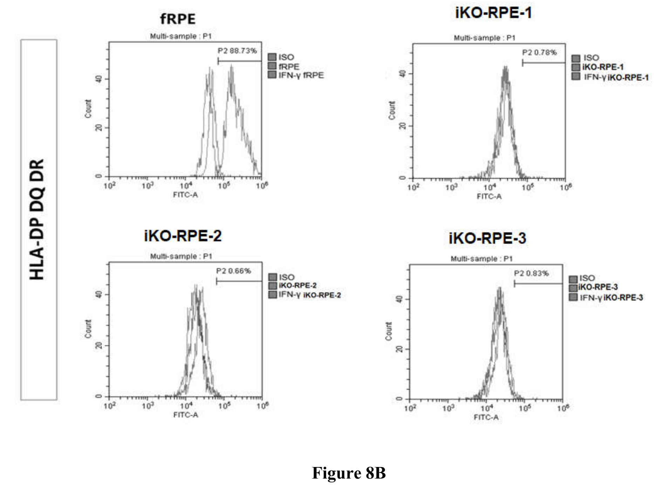

**Figure 8B** shows the assay results for class II immune-privileged characteristics of the HLA gene cluster encoding HLA-DR, HLA-DQ, and HLA-DP (HLA-DR DQ DP).

### DETAILED DESCRIPTION OF THE INVENTION

**Overview of Experimental Group Protocol.** (1) Culturing human induced pluripotent stem cells (hiPSCs): iPSC^{HLA-KO} cells with knocked-out B2M gene (encoding HLA class I molecules) and CIITA gene (a master regulator of HLA class II molecules) were passaged at a ratio of 1:8 in 12-well plates pre-coated with Laminin and cultured in a 37°C, 5% CO₂ incubator. Cell morphology was observed daily under a microscope, and the E8 medium was replaced with fresh medium every day. Passaging was performed every 3-4 days at a ratio of 1:8. During passaging, cells were treated with 0.25% EDTA (w/v) for 5-10 minutes, washed once with DPBS, then gently pipetting up and down with 1 mL of E8 medium by pipetting no more than 8 times. The collected cells were transferred to new Laminin-coated 12-well plates for continued culture. On the day following each passage, 10 µM Y-27632 was added to the culture medium. After four passages, the cells were induced to differentiate. The differentiation process is detailed in Figure 1A.

(2) Induction of universal human induced pluripotent stem cells (iPSC^{HLA-KO}) into human retinal pigment epithelial cells (iPSC^{HLA-KO}-RPE): When the cultured hiPSCs reached approximately 80% confluency, this time point was defined as Day (-1). The cells were then dissociated and passaged at a split ratio of 1:10 into Laminin-coated 12-well plates. The culture medium was replaced with E8 medium supplemented with 10 µM Y-27632. After one day of treatment, this time point was defined as Day (0).

From Day (0) to Day (2), the culture medium was replaced with the differentiation medium Neuroectoderm Medium (the first medium in the kit). Neuroectoderm Medium consisted of E6 basal medium supplemented with any combination of 10-100 ng/mL SB431542, 50-100 ng/mL LDN193183, and 10-50 mM IWR-1, to induce neuroectodermal cell differentiation.

From neuroectodermal induction until Day (6), the culture medium was replaced with the differentiation medium Retinal Progenitor Medium (the second medium in the kit), consisting of E6 basal medium supplemented with 1-50 mM Nicotinamide, to induce differentiation into RPE progenitor cells.

From completion of RPE progenitor induction until Day (10), the culture medium was replaced with the differentiation medium Immature RPE Medium (the third medium in the kit). Immature RPE Medium consisted of E6 basal medium supplemented with any combination of 10-200 ng/mL Activin A, 1-10 µM SU5402, and 1-10 µM CHIR99021, to further induce differentiation of RPE progenitor cells.

Upon completion of RPE progenitor cell induction to Passage 0 (Day 16), the differentiating cells were digested with 1× Tryple at 37°C in a 5% CO₂ incubator for 5-10 minutes for passaging. After passaging, the cells were plated onto Laminin-precoated 12-well plates. The resulting immature iPSC^{HLA-KO}-RPE cells were passaged at a split ratio of 1:(3-20) and cultured in a Mature RPE Medium (the fourth culture medium of the kit). The Mature RPE Medium primarily comprised an E6 basal medium supplemented with any combination of 1-50 mM nicotinamide and 1-10 µM CHIR99021.

The cells were further cultured in the Mature RPE Medium. At Day (16)-Passage 1 and Day (22)-Passage 2, the cells were digested with 1× Tryple in a 37°C, 5% CO₂ incubator for 5-10 minutes for passaging. The resulting more mature iPSC^{HLA-KO}-RPE cells were passaged at a ratio of 1:4 and continued to be cultured in the Mature RPE Medium until Day (30).

**Table 1. Small molecule selection scheme at each stage.**

| Group | Neuroectodermal Cell Induction Stage | RPE Progenitor Cell Induction Stage | RPE Cell Induction Stage | RPE Cell Maturation Stage |
|---|---|---|---|---|
| iKO-RPE-1 | 25µMSB431542 50nM LDN193183, 20µM IWR-1 | 25 mM Nicotinamide | 100ng/mL Activin A | 5µM CHIR9902, 25mM Nicotinamide |
| iKO-RPE-2 | 25µM SB431542, 20µM IWR-1 | 25 mM Nicotinamide | 100ng/mL Activin A, 5µM SU5402 | 5µM CHIR9902 |
| iKO-RPE-3 | 50nM LDN193183 | 25 mM Nicotinamide | 100ng/mL Activin A, 5µM SU5402, 5µM CHIR99021 | 5µM CHIR9902, 25mM Nicotinamide |

The applicant has verified through repeated experiments that SB431542 at 1-50 µM, LDN193183 at 10-100 nM, IWR-1 at 10-100 µM, Nicotinamide at 1-50 mM, Activin A at 10-200 ng/mL, CHIR99021 at 1-10 µM, and SU5402 at 1-10 µM, when applied within their respective ranges under the same signaling pathway conditions, all produce effects identical or similar to those shown in Table 1.

### Embodiments of the Invention

The following provides a clear and complete description of the technical solutions of the present application in conjunction with specific embodiments.

### 1. Definitions.

iPSC refers to induced pluripotent stem cells. hiPSC refers to human induced pluripotent stem cells, which are stem cells reprogrammed from human peripheral blood mononuclear cells, possessing self-renewal ability and the capacity to differentiate into the three germ layer lineages. Three germ layer differentiation refers to differentiation into ectodermal, mesodermal, and endodermal cell lineages. Source: T/CSCB0005-2021, Group Standard Human Induced Pluripotent Stem Cells, issued by the Chinese Society for Cell Biology.

Universal iPSC^{HLA-KO} refers to iPSCs in which class I genes (e.g., B2M) and/or class II genes (e.g., CIITA) of the HLA gene cluster are knocked out using CRISPR/Cas9 technology. The method of obtaining such cells may follow the steps disclosed in Chinese Patent Application No. 2022106036092, titled "Method for Preparing Hypoimmunogenic iPSCs, Hypoimmunogenic iPSCs, and Compositions Thereof," except that the iPSCs described herein do not rely on CD47 editing. Alternatively, hypoimmunogenic hPSCs can also be engineered through knockout of β-2 microglobulin (B2M) within the endogenous B2M locus or by biallelic knock-in of HLA-G1, thereby generating hPSCs in which classical human leukocyte antigen (HLA) class I proteins are not expressed on the cell surface. Reference: Shi L, Li W. et al., Generation of hypoimmunogenic human pluripotent stem cells via expression of membrane-bound and secreted β2m-HLA-G fusion proteins, Stem Cells. 2020 Nov;38(11):1423-1437. doi:10.1002/stem.3269. Epub 2020 Sep 15. PMID: 32930470.

RPE cells refer to retinal pigment epithelium (RPE) cells, which are located on the outer side of the retina as a densely pigmented epithelial monolayer situated between the choroid and the neural retina. RPE cells provide structural support, nutrition, and recycling functions for photoreceptor cells. In the present application, RPE cells are identified based on the following criteria: (i) positive expression of RPE-specific marker proteins TYR, RPE65, MITF, PAX6, and BEST1; (ii) the presence of melanin granules (brown-black pigmentation); and (iii) characteristic cellular morphology, namely tight intercellular junctions and a typical polygonal, cobblestone-like appearance. The functional properties of RPE cells are verified herein through in vitro phagocytosis assays.

iRPE refers to iPSC-RPE, induced pluripotent stem cell-derived retinal pigment epithelium, which are RPE cells derived from human induced pluripotent stem cells. The iRPE cells are cultured under adherent conditions, in which the cells are seeded onto Laminin-precoated plates for differentiation. The Laminin may be selected from Laminin 521, Laminin α4 antibody (Laminin α4), Laminin α5β1γ1, Laminin α1β1γ1, or the like; alternatively, Vitronectin (VTN) may be used as a substitute.

Signaling pathways: The development of cell lineages is generally regulated by multiple signaling pathways that control cell proliferation and differentiation. Each of these pathways is regulated by a series of complex genetic factors, epigenetic factors (such as histone modifications), and exogenous signal transduction factors, which can govern cell fate and behavior during development and differentiation. In the present disclosure, the regulated pathways include the TGF-β/Nodal/Activin signaling pathway, the BMP signaling pathway, the WNT signaling pathway, the Nicotinamide metabolic signaling pathway, and the FGF signaling pathway. Pathways not described herein are not further regulated with small molecule additives.

Neuroectodermal cell induction stage: At this stage, the cells reach 100% confluency, and the cell morphology adopts a neuroectodermal cell-like appearance.

RPE progenitor cell induction stage: At this stage, the cells are densely packed, with indistinct cell boundaries, irregular morphology, and cellular aggregation.

RPE cell induction stage: Approximately 10% of non-RPE cells undergo apoptosis, while the surviving cells develop into tightly packed, cobblestone-like, RPE-like cells in a monolayer, referred to as RPE precursor cells.

RPE cell maturation stage: The RPE precursor cells gradually develop into cobblestone-like structures with evenly distributed melanin. When functional monolayer RPE cells are formed, the cells exhibit a more regular cobblestone morphology and deeper melanin pigmentation.

A key indicator of "functionality" is the in vitro phagocytic activity of RPE cells. Phagocytosis occurs in three stages: binding, internalization, and degradation. During binding, the apical microvilli of the RPE cell membrane attach to shed photoreceptor outer segments, which are subsequently internalized into the cell, and finally transported by the cytoskeleton and vesicles to lysosomes for degradation.

TGF-β/Nodal/Activin signaling pathway: This refers to the pathway in which the TGF-β signaling pathway, the Nodal signaling pathway, and the Activin signaling pathway are simultaneously activated or inhibited.

Nicotinamide metabolic signaling pathway: This pathway is regulated by the small molecule Nicotinamide. The theoretical basis is described in: Hong S. et al., Nicotinamide N-methyltransferase regulates hepatic nutrient metabolism through Sirt1 protein stabilization. Nat Med. 2015 Aug;21(8):887-894. doi:10.1038/nm.3882. Epub 2015 Jul 13. PMID: 26168293; PMCID: PMC4529375.

The culture media used in the differentiation process described herein are serum-free media. During the iPSC culture stage, E8 medium is used; during the differentiation of iPSCs into RPE cells, E6 medium supplemented with small molecules is employed. E6 medium refers to a formulation derived from E8 medium with the omission of FGF2 and TGF-β components.

Unless otherwise specified, the reagents, materials, and equipment used in the present embodiment are commercially available, and the experimental methods are conventional methods well known in the art.

Activin A belongs to the transforming growth factor (TGF) superfamily and plays roles in early embryonic development, vascular smooth muscle cell proliferation, arteriosclerosis, neural differentiation, hematopoietic cell proliferation and differentiation, and regulation of pituitary hormone secretion in the endocrine system. In the present application, its function is to activate the TGF-β/Activin signaling pathway, thereby promoting the differentiation of retinal progenitor cells into RPE precursor cells.

In this specification, various chemical substances such as WNT inhibitors, BMP inhibitors, and FGF inhibitors include their free forms, salts, and the like. For example, Y-27632 includes free-form Y-27632 as well as its salts, such as Y-27632 hydrochloride and Y-27632 dihydrochloride.

The term "not more than" as used in the text refers to less than or equal to.

Day (0) refers to day 0, Day (1) refers to day 1, and so on.

fRPE refers to human fetal retinal pigment epithelial cells, which serve as the positive control for iRPE. These cells are obtained from the isolation and culture of eyeballs from aborted fetuses, using methods that are known in the art. The methods include, but are not limited to, those disclosed in Chinese Patent Application CN201310552005.0, entitled "Method for Isolation and Culture of Human Fetal Retinal Pigment Epithelial Cells."

**2. Culture condition design.** In all procedures described herein, adherent culture is employed for cell culture and differentiation. There is no particular limitation on the culture vessels used for adherent culture of human induced pluripotent stem cells or for their differentiation, so long as they permit adherent growth and enable the differentiation of human induced pluripotent stem cells into RPE cells in the culture medium. In the present application, the selected culture vessels are plates precoated with Laminin.

In the present application, the key feature is that by using only chemical small molecules in combination with a basal medium, RPE cells of high yield and stability can be obtained within a shorter time under simplified and well-defined medium conditions, while the differentiated RPE cells exhibit enhanced phagocytic function.

Accordingly, the medium employed in the present application is primarily based on E6 medium or other media with components equivalent to or comparable in function to those of E6. Of course, media with more complex components than E6 may also be used and are capable of achieving the technical effects of the present application. However, the primary objective of the present application is to establish a clear differentiation pathway; the secondary objective is to use a simplified and well-defined medium for differentiation so as to ensure subsequent industrial-scale production.

Medium replacement is performed at the end of each stage of the differentiation process. Specifically, this refers to replacing the medium used in the neuroectodermal cell induction stage with that used in the RPE progenitor cell induction stage, replacing the RPE progenitor medium with the RPE cell induction medium, or replacing the RPE cell induction medium with the RPE cell maturation medium.

In the present disclosure, during each stage-the neuroectodermal cell induction stage, the RPE progenitor cell induction stage, the RPE cell induction stage, and the RPE cell maturation stage-the culture medium is refreshed daily or every other day. "Refreshing the medium" refers to replacing it with the same basal medium supplemented with the same small molecules.

The culture conditions (such as culture temperature and CO₂ concentration) can be appropriately determined. Although the culture temperature is not particularly limited, it is generally 30°C to 40°C, preferably 37°C. The CO₂ concentration is about 1%-10%, preferably about 5%.

**3. Selection of iPSCs.** As the iPSCs used during the neuroectodermal cell induction stage, the iPSCs are preferably iPSCs or immune-privileged universal iPSC^{HLA-KO} lines with B2M and CIITA genes knocked out. Such universal iPSC^{HLA-KO} lines can be prepared by methods known to those skilled in the art, as described above. This avoids immune rejection of the differentiated RPE cells and renders them more universal. iPSCs may also be prepared by conventional methods well known in the art.

Compared with embryonic stem cells (ESCs, derived from the commercial ESC line H9), the use of iPSCs or universal iPSC^{HLA-KO} in the present application involves additional considerations during differentiation into RPE cells, such as the stability of pluripotency in universal iPSC^{HLA-KO} and whether their low immunogenicity can be effectively retained after differentiation. These factors also represent technical challenges in the differentiation process of the present application. To mitigate the impact of these issues on the stability of RPE cells differentiated from iPSCs, and to improve differentiation efficiency, the iPSCs or universal iPSC^{HLA-KO} are subjected to pretreatment. Specifically, the iPSCs or universal iPSC^{HLA-KO} are cultured in iPSC medium for 4-7 passages, preferably 4-5 passages. The objectives are to obtain more stable iPSCs or universal iPSC^{HLA-KO}, ensure higher purity by removing contaminating cells, achieve stable differentiation efficiency, and ensure that the differentiated RPE cells retain low immunogenicity.

The split ratio is 1:(3-12), preferably 1:(3-10), and exemplarily 1:3, 1:4, 1:5, 1:6, 1:7, 1:8, 1:9, or 1:10. When the ratio is less than 1:3, cells become over-confluent after passaging, leading to crowding and cell death. When the ratio exceeds 1:12, the cells tend to differentiate into other heterogeneous cell types. In practice, passaging at ratios within 1:(3-10) has minimal impact on the performance, differentiation efficiency, and stability of the resulting RPE cells. In the subsequent embodiments of the present application, a passaging ratio of 1:8 is used for verification.

The pluripotent stem cell culture medium may be selected from any commercially available stem cell medium. In the present application, to ensure defined components and reproducibility of the differentiation process, the pluripotent stem cell medium employed comprises a basal medium supplemented with Y-27632. The dosage of Y27632 should be sufficient to maintain adherent culture of pluripotent stem cells, and can be appropriately determined by those skilled in the art. For example, Y27632 is specifically used at 10 µM (10 µmol/L). As basal media for maintaining the undifferentiated state, commercially available E8, Stem Flex, or mTeSR1 may also be used. These media can support maintenance culture or expansion culture of pluripotent stem cells.

The iPSCs to be passaged were digested by treatment with 0.25% (w/v) EDTA for 5-10 minutes, followed by one wash with DPBS buffer, and then gently pipetting up and down with 1 mL of iPSC medium. The cells were subsequently reseeded into Laminin-precoated 12-well plates for continued culture. In this embodiment, EDTA, a chemically defined reagent, was used in place of proteases to ensure that each step of the differentiation pathway employed chemically well-defined components to delineate the differentiation pathways governing each stage of the differentiation process. The ultimate purpose is to avoid karyotypic alterations in cells that may occur due to protease treatment during digestion, thereby ensuring high stability and reproducibility in the subsequent differentiation process.

**4. Preparation of retinal pigment epithelium (RPE) cells.** Figure 1A illustrates the small molecule-induced differentiation process using E6 as the basal medium. Figure 1B shows bright-field images of typical cell morphology at different time points during the differentiation of iPSC-RPE or iPSC^{HLA-KO} -RPE cells.

The RPE cells of the present application are derived from iPSCs (referring collectively to iPSCs or universal iPSC^{HLA-KO}). Differentiation into RPE cells is achieved by supplementing E6 basal medium with chemical small molecules serving as inhibitors or activators, proceeding through the following stages: neuroectodermal cell induction stage, RPE progenitor cell induction stage, RPE cell induction stage, and RPE cell maturation stage.

The main process is as follows: (1) Day 0-Day 2, Neuroectodermal Cell Induction Stage (Neuroectoderm): Differentiation is induced by inhibiting any one or more of the following signaling pathways: the TGF-β/Nodal/Activin signaling pathway, the BMP signaling pathway, and the WNT signaling pathway.

Specifically, inhibition of the TGF-β/Nodal/Activin signaling pathway is carried out using small molecule inhibitors, including but not limited to SB431542. Preferably, SB431542 is added at a concentration of 1-50 µM. At concentrations that are too low, stem cells fail to differentiate toward the ectodermal lineage, while at excessively high concentrations, the cost becomes prohibitive. The concentration may be maintained at a constant level each day or varied across days, provided that RPE cells can be obtained by the method of the present application.

Inhibition of the BMP signaling pathway is carried out using small molecule inhibitors, including but not limited to LDN193183, at a concentration of 10-100 nM. At concentrations that are too low, stem cells fail to differentiate toward the ectodermal lineage, while at excessively high concentrations, the cost becomes prohibitive. The concentration may be maintained at a constant level each day or varied across days, provided that RPE cells can be obtained by the method of the present application.

Inhibition of the WNT signaling pathway is primarily achieved using small molecule inhibitors, including but not limited to IWR-1 or IWR-2. The concentration ranges are 10-100 µM for IWR-1 and 10-100 µM for IWR-2. At concentrations that are too low, stem cells differentiate toward the mesodermal lineage, while at excessively high concentrations, the cost becomes prohibitive. In practice, the concentration may be maintained at a constant level each day or varied across days, provided that RPE cells can be obtained by the method of the present application.

The duration of the neuroectodermal cell induction stage is not particularly limited, generally not more than 5 days, and preferably not more than 3 days.

As shown in FIG. 1B, the endpoint of the neuroectodermal cell induction stage is defined as the cells reaching 100% confluency with morphology consistent with neuroectodermal cell-like appearance. At this point, differentiation is induced into the RPE progenitor cell induction stage.

(2) Day 2-Day 6, RPE Progenitor Cell Induction Stage: Differentiation is induced by activating the Nicotinamide metabolic signaling pathway.

Specifically, inhibition of the activation of the Nicotinamide metabolic signaling pathway is achieved through the use of small molecules, including but not limited to Nicotinamide. The concentration of Nicotinamide is selected to be 1-50 mM. At concentrations that are too low, the cells fail to differentiate into retinal progenitor cells, while at excessively high concentrations, massive cell death occurs and the cost becomes prohibitive. The concentration may be maintained at a constant level each day or varied across days, provided that RPE cells can be obtained by the method of the present application.

The duration of the RPE progenitor cell induction stage is not particularly limited, generally not more than 6 days, and preferably not more than 4 days.

As shown in Figure 1B, the RPE progenitor cell induction stage may be characterized by cellular morphology, or alternatively, by certain experimental indicators used to determine the completion of this process.

(3) Day 6-Day 10, RPE Cell Induction Stage (RPE specialization): Differentiation is induced by any one or more of the following signaling pathways: activating the TGF-β/Nodal/Activin signaling pathway, inhibiting the FGF signaling pathway, and activating the WNT signaling pathway.

Specifically, inhibition of the FGF signaling pathway in this process is achieved through the use of small molecules, including but not limited to SU5402, at a concentration of 1-10 µM. At concentrations that are too low, the resulting RPE precursor cells are impure, with many neural cells appearing; at excessively high concentrations, the cells die. Activation of the TGF-β/Nodal/Activin signaling pathway in this process is achieved through the use of small molecules, including but not limited to Activin A, at a concentration of 10-200 ng/mL. At concentrations that are too low, the resulting RPE precursor cells are impure, with many neural cells appearing; at excessively high concentrations, the cost becomes prohibitive. Activation of the WNT signaling pathway in this process is achieved through the use of small molecules, including but not limited to CHIR99021, at a concentration of 1-10 µM. At concentrations that are too low, the resulting RPE precursor cells are impure, with many neural cells appearing; at excessively high concentrations, the cells exhibit fibroblast-like morphology.

The duration of the RPE cell induction stage is not particularly limited, generally not more than 10 days, and preferably not more than 6 days. The term "not more than" refers to less than or equal to.

As shown in Figure 1B, the endpoint of the RPE cell induction stage is characterized by apoptosis of approximately 10% of non-RPE cells, while the surviving cells develop into tightly packed, cobblestone-like RPE-like cells in a monolayer, which are defined as RPE precursor cells.

(4) Day 10-Day 30, RPE Cell Maturation Stage (RPE maturation): Differentiation is induced by activating any one or more of the following signaling pathways: the Nicotinamide metabolic signaling pathway and the WNT signaling pathway.

Specifically, the late-stage RPE culture medium comprises E6 basal medium supplemented with small molecules that activate the Nicotinamide metabolic signaling pathway and the WNT signaling pathway. The small molecule used to activate the Nicotinamide metabolic signaling pathway is Nicotinamide, at a concentration of 1-50 mM. At concentrations that are too low, RPE cell maturation is slowed, affecting the maturation timeline; at excessively high concentrations, the cost becomes prohibitive. The small molecule used to activate the WNT signaling pathway is CHIR99021, at a concentration of 1-10 µM. At concentrations that are too low, RPE cell maturation is slowed, affecting both maturation time and RPE cell proliferation; at excessively high concentrations, the cost becomes prohibitive.

The duration of the RPE cell maturation stage is not particularly limited, generally not more than 15 days, and preferably not more than 10 days.

As shown in Figure 1B, during the RPE cell maturation stage, RPE precursor cells gradually develop into cobblestone-like structures with evenly distributed melanin. When functional monolayer RPE cells are formed, the cells exhibit a more regular cobblestone-like morphology with intensified melanin pigmentation.

In the differentiation method of the present application, there is no need for manual removal of non-RPE-like heterogeneous cells during the differentiation process. Pure iPSC-RPE (iPSC-RPE or iPSC^{HLA-KO}-RPE) cells can be obtained during the RPE cell maturation stage simply through passaging culture. The basal component of the differentiation medium at this stage comprises only E6 medium (which differs from E8 medium by the absence of bFGF and TGF-β) and contains no animal-derived components. At the same time, by regulating the signaling pathways as disclosed herein, the RPE cells differentiated from iPSCs (iPSCs or universal iPSC^{HLA-KO}) achieve a yield up to approximately 10¹¹ times higher than the initial seeding of iPSCs.

Compared with differentiation processes disclosed in the prior art, such as: Plaza Reyes, A. et al.. (2020) (Reference 1), in which the published differentiation method requires nearly 60 days to obtain RPE cells with uniform pigmentation, and Regent, F., et al., (2019) (Reference 2), in which 42 days are required to obtain stable RPE cells, the present application achieves uniformly pigmented RPE cells in only about 30 days.

Preferably, in order to obtain a greater yield of RPE cells and to ensure the functional stability of the RPE cells, the passaging ratio during the RPE cell induction stage and the RPE cell maturation stage is 1:(3-20). Exemplary ratios include 1:3, 1:4, 1:5, 1:6, 1:7, 1:8, 1:9, 1:10, 1:11, 1:12, 1:13, 1:14, 1:15, 1:16, 1:17, 1:18, 1:19, or 1:20.

In the present application, verification of the differentiation efficiency of iPSCs into RPE cells (iPSCs or universal iPSC^{HLA-KO}) and functional characterization of the RPE cells is primarily conducted by flow cytometry, immunofluorescence, reverse transcription PCR (RT-PCR), and in vitro phagocytosis assays.

Embodiment 1. **Validation of different differentiation methods using the same** cells (iPSC^{HLA-KO}). Overview of the experimental group protocol. (1) Culture of human induced pluripotent stem cells (hiPSCs): iPSC^{HLA-KO} cells, in which the HLA class I gene B2M and the master regulator of HLA class II, CIITA, were knocked out, were seeded at a ratio of 1:8 into Laminin-precoated 12-well plates and cultured in a 37°C, 5% CO₂ incubator. Cell morphology was observed daily under a microscope, and the E8 medium was replaced with fresh medium every day. Passaging was performed every 3-4 days at a ratio of 1:8. For passaging, the cells were treated with 0.25% EDTA (w/v) for 5-10 minutes, washed once with DPBS buffer, and gentle pipetting up and down in 1 mL of E8 medium by pipetting no more than 8 times. The collected cells were then reseeded into new Laminin-precoated 12-well plates for continued culture. On the day following each passage, 10 µM Y-27632 was added to the culture medium. After four passages, the cells were subjected to differentiation, with the specific differentiation process shown in Figure 1A.

(2) Induction of universal human induced pluripotent stem cells (iPSC^{HLA-KO}) into human retinal pigment epithelial cells (iPSC ^{HLA-KO}-RPE): When the cultured hiPSCs reached 80% confluency, this time point was defined as Day (-1). At this stage, the cells were digested and passaged at a 1:10 ratio into Laminin-precoated 12-well plates. The culture medium was replaced with E8 medium supplemented with 10 µM Y-27632. After one day of treatment, this time point was defined as Day (0).

From Day (0) to Day (2), the culture medium was replaced with the differentiation medium Neuroectoderm Medium (the first medium in the kit). Neuroectoderm Medium consisted of E6 basal medium supplemented with any combination of 10-100 ng/mL SB431542, 50-100 ng/mL LDN193183, and 10-50 mM IWR-1, to induce neuroectodermal cell differentiation.

Neuroectodermal induction was continued until Day (6), at which point the culture medium was replaced with the differentiation medium Retinal Progenitor Medium (the second medium in the kit). Retinal Progenitor Medium consisted of E6 basal medium supplemented with 1-50 mM Nicotinamide to induce RPE progenitor cells.

Upon completion of RPE progenitor induction up to Day (10), the culture medium was replaced with the differentiation medium Immature RPE Medium (the third medium in the kit). Immature RPE Medium consisted of E6 basal medium supplemented with any combination of 10-200 ng/mL Activin A, 1-10 µM SU5402, and 1-10 µM CHIR99021, to induce RPE progenitor cells.

Upon completion of RPE progenitor induction up to Passage 0 (Day 16), the differentiating cells were digested with 1× Tryple in a 37°C, 5% CO₂ incubator for 5-10 minutes for passaging. The subsequent 12-well plates used for culture were precoated with Laminin. The resulting immature iPSC ^{HLA-KO}-RPE cells were passaged at a ratio of 1:(3-20) and cultured in Mature RPE Medium (the fourth medium in the kit). Mature RPE Medium consisted primarily of E6 basal medium supplemented with any combination of 1-50 mM Nicotinamide and 1-10 µM CHIR99021.

The cells were further cultured in Mature RPE Medium. At Day (16)-Passage 1 and Day (22)-Passage 2, the cells were digested with 1× Tryple in a 37°C, 5% CO₂ incubator for 5-10 minutes for passaging. The resulting more mature iPSC ^{HLA-KO}-RPE cells were passaged at a ratio of 1:4 and continued to be cultured in Mature RPE Medium until Day (30).

**Table 1. Small molecule selection scheme at each stage**

| Group | Neuroectodermal Cell Induction Stage | RPE Progenitor Cell Induction Stage | RPE Cell Induction Stage | RPE Cell Maturation Stage |
|---|---|---|---|---|
| iKO-RPE-1 | 25µM SB431542, 50nM LDN193183, 20µM IWR-1 | 25mM Nicotinamide | 100ng/mL Activin A | 5µM CHIR9902, |
| | | | | 25mM Nicotinamide |
| iKO-RPE-2 | 25µM SB431542, 20µM IWR-1 | 25mM Nicotinamide | 100ng/mL Activin A, 5µM SU5402 | 5µM CHIR9902 |
| iKO-RPE-3 | 50nM LDN193183 | 25mM Nicotinamide | 100ng/mL Activin A, 5µM SU5402, 5µM CHIR99021 | 5µM CHIR9902, |
| | | | | 25mM Nicotinamide |

The applicant has verified through repeated experiments that SB431542 at 1-50 µM, LDN193183 at 10-100 nM, IWR-1 at 10-100 µM, Nicotinamide at 1-50 mM, Activin A at 10-200 ng/mL, CHIR99021 at 1-10 µM, and SU5402 at 1-10 µM, when applied within their respective ranges under the same signaling pathway conditions, all produce effects identical or similar to those shown in Table 1.

Embodiment 2. **Validation of different differentiation methods using the same cells (conventional iPSCs).** The only difference from Embodiment 1 is that the cells selected were conventional iPSCs.

**Table 2. Small molecule selection scheme at each stage**

| Group | Neuroectodermal Cell Induction Stage | RPE Progenitor Cell Induction Stage | RPE Cell Induction Stage | RPE Cell Maturation Stage |
|---|---|---|---|---|
| iRPE-1 | 25µM SB431542, 50nM LDN193183, 20µM IWR-1 | 25mM Nicotinamide | 100ng/mL Activin A | 5µM CHIR9902, 25mM Nicotinamide |
| iRPE-2 | 25µM SB431542, 20µM IWR-1 | 25mM Nicotinamide | 100ng/mL Activin A, 5µM SU5402 | 5µM CHIR9902 |
| iRPE-3 | 50nM LDN193183 | 25mM Nicotinamide | 100ng/mL Activin A, 5µM SU5402, 5µM CHIR99021 | 5µM CHIR9902, 25mM Nicotinamide |

The applicant has verified through repeated experiments that SB431542 at 1-50 µM, LDN193183 at 10-100 nM, IWR-1 at 10-100 µM, Nicotinamide at 1-50 mM, Activin A at 10-200 ng/mL, CHIR99021 at 1-10 µM, and SU5402 at 1-10 µM, when applied within their respective ranges under the same signaling pathway conditions, all produce effects identical or similar to those shown in Table 2.

**3. Immunofluorescence assay.** When the cells of each experimental group, the positive control group, and the negative control group reached confluency, the supernatant was discarded, and the cells were washed once with DPBS. A 4% Paraformaldehyde solution (v/v) was then added, and the cells were incubated at room temperature for 15 minutes. Afterward, the cells were washed three times with DPBS, each wash lasting 2 minutes. The cells were then permeabilized with DPBS containing 0.2% Triton-X100 (w/v) for 10 minutes, and blocked with DPBS containing 1% bovine serum albumin (BSA, w/v) at room temperature for 1 hour. After discarding the BSA solution without washing, the cells were directly incubated with primary antibodies at 4°C overnight: mouse monoclonal anti-MITF antibody (diluted 1:100), mouse monoclonal anti-OCT2 antibody (diluted 1:300), rabbit monoclonal anti-TYROSINASE antibody (diluted 1:100), rabbit monoclonal anti-RPE65 antibody (diluted 1:100), and rabbit monoclonal anti-ZO-1 antibody (diluted 1:500). The cells were then washed three times with PBS, each wash lasting 5 minutes. Secondary antibodies were subsequently added and incubated at room temperature for 2 hours: Alexa Fluor 546-conjugated goat anti-rabbit IgG (diluted 1:1000), Alexa Fluor 546-conjugated goat anti-mouse IgG (diluted 1:1000), FITC-conjugated goat anti-rabbit IgG (diluted 1:1000), and FITC-conjugated goat anti-mouse IgG (diluted 1:1000). After three washes with DPBS, each lasting 5 minutes, the cells were incubated with DAPI staining solution at room temperature in the dark for 15 minutes. Finally, the cells were washed three more times with DPBS, each wash lasting 5 minutes, and observed and imaged using an inverted fluorescence microscope.

Immunofluorescence identification was performed on the differentiated target cells, iRPE or iKO-RPE. The results showed that both iRPE and iKO-RPE cells obtained through passaging culture positively expressed RPE cell-specific marker proteins, including MITF, ZO-1, TYROSINASE (TYR), BEST1, and RPE65, as shown in Figure 2. Figure 2A shows immunofluorescence identification of specific protein expression in differentiated iKO-RPE cells and the positive control fRPE cells. Figure 2B shows immunofluorescence identification of specific protein expressions in differentiated iRPE cells.

**4. Reverse transcription quantitative PCR (RT-qPCR) assay.** Total RNA was extracted from cells of each group using an RNA extraction kit (Vazyme, China), and the OD value was measured to ensure that the OD260/OD280 ratio of RNA samples from each group was between 1.8 and 2.1, thereby confirming RNA purity. The extracted RNA was then reverse transcribed into cDNA using a reverse transcription kit (Vazyme, China), with the reaction system and program performed according to the manufacturer's instructions. The resulting cDNA was used for RT-PCR, with primer sequences listed in Table 3. The reaction system followed the instructions of the SYBR kit (Bio, USA). After a brief centrifugation, reactions were run under the following conditions: pre-denaturation at 95°C for 5 minutes, followed by 40 cycles of 94°C for 30 seconds, 59°C for 30 seconds, and 72°C for 30 seconds. For each reaction, 2 µL of product was mixed with 3 µL of SYBR Green Mix and analyzed.

**Table 3. Primer sequences**

| Primer | Sequence (5' to 3') |
|---|---|
| GAPDH-F | GGAGCGAGATCCCTCCAAAAT |
| GAPDH-R | GGCTGTTGTCATACTTCTCATGG |
| RPE65-F | TTGGATCTGAGCCATTTTACCAC |
| RPE65-R | GTCAGTAACCTCTACTCCTCGAA |
| MITF-F | TGCCCAGGCATGAACACAC |
| MITF-R | TGGGAAAAATACACGCTGTGAG |
| PAX6-F | CCAGGGCAATCGGTGGTAGT |
| PAX6-R | ACGGGCACTCCCGCTTATAC |
| BEST1-F | AACTGAGCCTACCACACAACA |
| BEST1-R | CGGATTCGACCTCCAAGCC |

| | |
|---|---|
| Note: F = Forward primer; R = Reverse primer. The product sequence lengths corresponding to the primers are as follows: GAPDH, 197 bp; RPE65, 236 bp; MITF, 276 bp; PAX6, 84 bp; and BEST1, 88 bp. | |

The expression levels of RPE65, MITF, PAX6, and BEST1 were used to assess RPE expression, with GAPDH serving as the internal reference gene to normalize and standardize the expression of the target genes.

RT-PCR results also demonstrated that the differentiated RPE cells positively expressed RPE-specific marker genes, including RPE65, MITF, PAX6, and BEST1. These RPE marker genes were positively expressed in the positive control hRPE cells, but negatively expressed in the negative control hiPSCs (FIG. 3). Figure 3 shows RT-PCR analysis of specific gene expression in iRPE, iKO-RPE, and the positive control fRPE: Figure 3A shows the expression of PAX6 in iPSC, fRPE, iKO-RPE-1, iKO-RPE-2, iKO-RPE-3, iRPE-1, iRPE-2, and iRPE-3; Figure 3B shows the expression of MITF in iPSC, fRPE, iKO-RPE-1, iKO-RPE-2, iKO-RPE-3, iRPE-1, iRPE-2, and iRPE-3; FIG. 3C shows the expression of RPE cell RPE65 in iPSC, fRPE, iKO-RPE-1, iKO-RPE-2, iKO-RPE-3, iRPE-1, iRPE-2, and iRPE-3; and Figure 3D shows the expression of BEST1 in iPSC, fRPE, iKO-RPE-1, iKO-RPE-2, iKO-RPE-3, iRPE-1, iRPE-2, and iRPE-3. In the figures, "Normalized to GAPDH" refers to expression levels relative to GAPDH.

**5. Flow cytometry assay.** When the cultured cells reached confluency, they were digested into a single-cell suspension and washed once with DPBS. The cells were then fixed with 4% paraformaldehyde (v/v) at room temperature for 10 minutes, followed by one wash with DPBS. After centrifugation and discarding the supernatant, the cells were permeabilized with 0.2% Triton-X100 (w/v) for 10 minutes, washed once with DPBS, centrifuged again, and then blocked with DPBS containing 1% bovine serum albumin (BSA, w/v) at room temperature for 30 minutes. After centrifugation and removal of the BSA, for detection of PAX6, APC-conjugated anti-PAX6 antibody was added, and the cells were incubated at room temperature for 0.5 hours, followed by one wash with PBS. The supernatant was discarded, and the cells were resuspended in DPBS for flow cytometry analysis. For detection of MITF and RPE65, after removal of the BSA solution, the cells were directly incubated with primary antibodies without washing: mouse monoclonal anti-MITF antibody (diluted 1:100) and rabbit monoclonal anti-RPE65 antibody (diluted 1:100), overnight at 4°C. The next day, the cells were washed once with PBS, followed by incubation at room temperature for 1 hour with secondary antibodies: FITC-conjugated goat anti-mouse IgG (diluted 1:1000) and PE-conjugated goat anti-rabbit IgG (diluted 1:1000). After one wash with PBS and removal of the supernatant, the cells were resuspended in DPBS and analyzed using a flow cytometry system.

HiPSC-derived RPE (hiPSC-RPE) cells positively expressed RPE cell-specific marker proteins. The proportions of RPE65-, MITF-, and PAX6-positive cells were all greater than 97%, indicating that the purity of hiPSC-RPE was high and consistent with that of fRPE. Residual stem cell testing was also performed on the differentiated RPE cells. No stem cell residues were detected by either qPCR or flow cytometry.

Specifically, Figure 4A shows flow cytometry analysis of MITF expression in fRPE, iKO-RPE-1, iKO-RPE-2, and iKO-RPE-3 cells: (a) MITF expression in fRPE cells (98.98%); (b) MITF expression in iKO-RPE-1 cells (98.52%); (c) MITF expression in iKO-RPE-2 cells (99.41%); and (d) MITF expression in iKO-RPE-3 cells (99.26%). iKO-RPE-1, iKO-RPE-2, and iKO-RPE-3 were obtained according to three different experimental methods in Embodiment 1. Figure 4B shows flow cytometry analysis of MITF expression in iPSC-RPE cells: (a) MITF expression in iRPE-1 cells (98.56%); (b) MITF expression in iRPE-2 cells (97.93%); and (c) MITF expression in iRPE-3 cells (99.02%). iRPE-1, iRPE-2, and iRPE-3 were obtained according to three different experimental methods in Embodiment 2.

Figure 5A shows flow cytometry analysis of RPE65 expression in iPSC ^{HLA-KO}-RPE cells and the positive control fRPE: (a) RPE65 expression in fRPE cells (99.19%); (b) RPE65 expression in iKO-RPE-1 cells (99.02%); (c) RPE65 expression in iKO-RPE-2 cells (99.13%); and (d) RPE65 expression in iKO-RPE-3 cells (99.59%). iKO-RPE-1, iKO-RPE-2, and iKO-RPE-3 were obtained from iKO-RPE cells according to three different experimental methods described in Embodiment 1. Figure 5B shows flow cytometry analysis of RPE65 expression in iPSC-RPE cells: (a) RPE65 expression in iRPE-1 cells (99.54%); (b) RPE65 expression in iRPE-2 cells (99.83%); and (c) RPE65 expression in iRPE-3 cells (99.87%). iRPE-1, iRPE-2, and iRPE-3 were obtained according to three different experimental methods described in Embodiment 2.

Figure 6A shows flow cytometry analysis of PAX6 expression in iPSC ^{HLA-KO}-RPE cells and the positive control fRPE: (a) PAX6 expression in fRPE cells (99.10%); (b) PAX6 expression in iKO-RPE-1 cells (99.23%); (c) PAX6 expression in iKO-RPE-2 cells (99.37%); and (d) PAX6 expression in iKO-RPE-3 cells (98.18%). iKO-RPE-1, iKO-RPE-2, and iKO-RPE-3 were obtained according to three different experimental methods described in Embodiment 1. Figure 6B shows flow cytometry analysis of PAX6 expression in iPSC-RPE cells: (a) PAX6 expression in iRPE-1 cells (98.23%); (b) PAX6 expression in iRPE-2 cells (97.01%); and (c) PAX6 expression in iRPE-3 cells (98.65%). iRPE-1, iRPE-2, and iRPE-3 were obtained according to three different experimental methods described in Embodiment 2.

### 6. In vitro phagocytosis assay.

Each experimental group was seeded into 12-well plates at a density of 1×10⁶ cells per well and cultured at 37°C for 2 days. After 2 days, fluorescent particles were added, and the cells were incubated at either 4°C/ 37°C for 8 hours. Trypan Blue was then added to quench fluorescence. The cells were washed three times with DPBS, digested with Tryple×1, resuspended in DPBS, and subsequently analyzed using a flow cytometry system. The method was based on: Parinot C., Rieu Q., Chatagnon J., Finnemann S.C., Nandrot E.F. Large-scale purification of porcine or bovine photoreceptor outer segments for phagocytosis assays on retinal pigment epithelial cells. J Vis Exp. 2014 Dec 12;(94):52100. doi: 10.3791/52100. PMID: 25548986; PMCID: PMC4396958.

Another important function of RPE cells is the phagocytosis of shed photoreceptor outer segments. To evaluate the phagocytic function of differentiated RPE cells, fluorescent particles were co-incubated with the cells and subsequently traced. The results showed that flow cytometry provided a more intuitive visualization of fluorescent particles being phagocytosed into iPSC-derived RPE (iPSCs-RPE) cells and fRPE cells. Each experiment was repeated three times. Figure 7A shows the phagocytosis assay results for fRPE, iRPE-1, iRPE-2, iRPE-3, iKO-RPE-1, iKO-RPE-2, and iKO-RPE-3 at 37°C. Figure 7B shows the phagocytosis assay results for fRPE, iRPE-1, iRPE-2, iRPE-3, iKO-RPE-1, iKO-RPE-2, and iKO-RPE-3 at 4°C. Figure 7C shows the negative control group for fRPE, iRPE-1, iRPE-2, iRPE-3, iKO-RPE-1, iKO-RPE-2, and iKO-RPE-3, in which no fluorescent particles were added during incubation. In the figures, iKO-RPE refers to RPE cells differentiated from iPSC^{HLA-KO} (iPSC^{HLA-KO}-RPE), and iRPE refers to RPE cells differentiated from iPSCs (iPSC-RPE).

### 7. In vitro immunological function assay of iKO-RPE.

Abnormal expression of IFN-γ is associated with many autoinflammatory and autoimmune diseases. The importance of IFN-γ in the immune system lies primarily in its immunostimulatory and immunomodulatory functions. IFN-γ is secreted mainly by NK cells and NKT cells, where it participates in innate immunity; during antigen-specific immune responses, it is secreted by CD4⁺ TH1 cells and CD8 cytotoxic T cells. Under various pathological conditions, including infection, autoimmune diseases, transplant rejection, and allergic reactions, IFN-γ can serve as a disease marker. In the present application, an in vivo inflammatory environment was simulated under IFN-γ stimulation. Following knockout of the B2M and CIITA genes, the differentiated iKO-RPE cells exhibited inactivation of both HLA class I gene clusters MHC (e.g., HLA-A, HLA-B, HLA-C) and HLA class II gene clusters MHC (e.g., HLA-DR, HLA-DQ, HLA-DP), thereby eliminating immune rejection responses in vivo. The method was based on: Petrus-Reurer S., Winblad N., Kumar P., Gorchs L., Chrobok M., Wagner A.K., Bartuma H., Lardner E., Aronsson M., Plaza Reyes Á., André H., Alici E., Kaipe H., Kvanta A., Lanner F. Generation of Retinal Pigment Epithelial Cells Derived from Human Embryonic Stem Cells Lacking Human Leukocyte Antigen Class I and II. Stem Cell Reports. 2020 Apr 14;14(4):648-662. doi: 10.1016/j.stemcr.2020.02.006. Epub 2020 Mar 19. PMID: 32197113; PMCID: PMC7160308.

Specifically, each experimental group was seeded into 12-well plates at a density of 1×10⁵ cells per well and cultured at 37°C for 2 days and 5 days under conditions with or without IFN-γ stimulation. After 2 days, flow cytometry was performed to detect HLA class I proteins (HLA-A/B/C). After 5 days, flow cytometry was performed to detect HLA class II proteins (HLA-DP/DQ/DR). Flow cytometry results clearly showed that, under IFN-γ stimulation, iKO-RPE cells differentiated after knockout of the B2M and CIITA genes exhibited inactivation of HLA class I MHC (see Figure 8A) and HLA class II MHC (see Figure 8B).

An RPE cell preparation, comprising the pluripotent stem cell-derived RPE cell as described above.

Another object of the present application is to provide a pharmaceutical composition for ophthalmology, comprising the pluripotent stem cell-derived RPE cell as described above as an active ingredient.

Another object of the present application is to provide a reagent for evaluating the toxicity or efficacy of a test substance, comprising the pluripotent stem cell-derived RPE cell as described above.

Another object of the present application is to provide a method for evaluating the toxicity or efficacy of a test substance, comprising contacting the pluripotent stem cell-derived RPE cell as described above with the substance, and determining the effect of the substance on the cell.

The schemes described in Embodiment 1 and Embodiment 2 of the present application may also be implemented by means of a kit.

A kit comprises a culture medium combination, the culture medium combination including: a first culture medium, a second culture medium, a third culture medium, and a fourth culture medium.

The first culture medium comprises a basal medium and additive A; additive A is one or more selected from SB431542, LDN193183, IWR-1, or IWR-2 in any molar ratio; wherein SB431542 is added at 1-50 µM, LDN193183 is added at 10-100 nM, IWR-1 is added at 10-100 µM, and IWR-2 is added at 10-100 µM. The second culture medium comprises a basal medium and additive B; additive B is Nicotinamide, added at 1-50 mM.

The third culture medium comprises a basal medium and additive C; additive C is one or more selected from Activin A, SU5402, or CHIR99021 in any molar ratio; wherein Activin A is added at 10-200 ng/mL, SU5402 is added at 1-10 µM, and CHIR99021 is added at 1-10 µM.

The fourth culture medium comprises a basal medium and additive D; additive D is one or both of Nicotinamide or CHIR99021 in any molar ratio; wherein nicotinamide is added at 1-50 mM, and CHIR99021 is added at 1-10 µM.

As an improved technical solution of the present application, the basal medium is E6.

Implementation of the schemes described in Embodiment 1 and Embodiment 2 of the present application using the kit comprises the following steps:
Step 1: Pluripotent stem cells are cultured in the first culture medium for 2 days to obtain neuroectodermal cells.
Step 2: The neuroectodermal cells are cultured in the second culture medium for 4 days to obtain RPE progenitor cells.
Step 3: The RPE progenitor cells are cultured in the third culture medium for 4 days to obtain RPE cells.
Step 4: The RPE cells are cultured in the fourth culture medium for 20 days to obtain mature RPE cells.

### Industrial Applicability

In the present application, the differentiation process is combined with the in vivo differentiation signaling pathways of RPE cells, thereby clarifying the cellular states during differentiation, greatly improving differentiation efficiency and shortening the differentiation period. The process requires only 30 days, with high cell purity and high yield.

In the differentiation process of the present application, the required signaling pathway activators or inhibitors to be added are clearly defined, rendering the differentiation method fixed, highly efficient, and reproducible.

The differentiation process of the present application does not introduce any animal-derived components, thereby preventing the possibility of exogenous viral invasion and enhancing the safety of future clinical applications.

### REFRENCES

1. Plaza Reyes, A. et al. Identification of cell surface markers and establishment of monolayer differentiation to retinal pigment epithelial cells. Nat Commun 11, 1609 (2020));
2. Regent, F., Morizur, L., Lesueur, L. et al. Automation of human pluripotent stem cell differentiation toward retinal pigment epithelial cells for large-scale productions. Sci Rep 9, 10646 (2019)).
3. Chinese Patent CN110573610A, titled "Method for Preparing Retinal Pigment Epithelial Cells," application filed by Sumitomo Dainippon Pharma Co Ltd, Healios KK on March 8, 2018.
4. Chinese Patent Application No. CN202210603609.2, titled "Method for Preparing Hypoimmunogenic iPSCs, Hypoimmunogenic iPSCs, and Compositions Thereof," filed by Help Stem Cell Innovations Co Ltd on May 31, 2022.
5. Shi L, Li W. et al., Generation of hypoimmunogenic human pluripotent stem cells via expression of membrane-bound and secreted β2m-HLA-G fusion proteins, Stem Cells. 2020 Nov;38(11):1423-1437. doi:10.1002/stem.3269. Epub 2020 Sep 15. PMID: 32930470.
6. Hong S. et al., Nicotinamide N-methyltransferase regulates hepatic nutrient metabolism through Sirt1 protein stabilization. Nat Med. 2015 Aug;21(8):887-894. doi:10.1038/nm.3882. Epub 2015 Jul 13. PMID: 26168293; PMCID: PMC4529375.
7. Chinese Patent Application CN201310552005.0, entitled "Method for Isolation and Culture of Human Fetal Retinal Pigment Epithelial Cells," filed by Suzhou Ruiqi Biological Medicine Science & Technology Co Ltd on November 11, 2013.
8. Parinot C., Rieu Q., Chatagnon J., Finnemann S.C., Nandrot E.F. Large-scale purification of porcine or bovine photoreceptor outer segments for phagocytosis assays on retinal pigment epithelial cells. J Vis Exp. 2014 Dec 12;(94):52100. doi: 10.3791/52100. PMID: 25548986; PMCID: PMC4396958.
9. Petrus-Reurer S., Winblad N., Kumar P., Gorchs L., Chrobok M., Wagner A.K., Bartuma H., Lardner E., Aronsson M., Plaza Reyes Á., André H., Alici E., Kaipe H., Kvanta A., Lanner F. Generation of Retinal Pigment Epithelial Cells Derived from Human Embryonic Stem Cells Lacking Human Leukocyte Antigen Class I and II. Stem Cell Reports. 2020 Apr 14;14(4):648-662. doi: 10.1016/j.stemcr.2020.02.006. Epub 2020 Mar 19. PMID: 32197113; PMCID: PMC7160308.

## Claims

1. A pluripotent stem cell-derived RPE cell, wherein said RPE cell is obtained from pluripotent stem cell through the following differentiation steps:
a neuroectodermal cell induction stage, comprising inducing differentiation by inhibiting one or more signaling pathways selected from TGF-β/Nodal/Activin signaling pathway, BMP signaling pathway, and WNT signaling pathway;
an RPE progenitor cell induction stage, comprising inducing differentiation by activating the Nicotinamide metabolic signaling pathway;
an RPE cell induction stage, comprising inducing differentiation by one or more of activating TGF-β/Nodal/Activin signaling pathway, inhibiting FGF signaling pathway, and activating WNT signaling pathway; and
an RPE cell maturation stage, comprising inducing differentiation by activating one or more signaling pathway selected from Nicotinamide metabolic signaling pathway and WNT signaling pathway.

2. The pluripotent stem cell-derived RPE cell of claim 1, wherein said pluripotent stem cell is differentiated from ESC, iPSC, or universal iPSC^{HLA-KO}; said universal iPSC^{HLA-KO} is an iPSC with immune-privileged properties obtained by gene editing to specifically reduce HLA expression.

3. The pluripotent stem cell-derived RPE cell of claim 2, wherein said ESC is an ESC that has been passaged to 4-7 generations, and the split ratio for each passaging is 1:(3-12); the cultured ESC is digested into single cells and cultured in iPSC medium for one day, and then differentiated into RPE cells; said iPSC or said universal iPSC^{HLA-KO} is an iPSC that has been passaged to 4-7 generations, and the split ratio for each passaging is 1:(3-12); the cultured iPSC is digested into single cells and cultured in iPSC medium for one day, and then differentiated into RPE cells.

4. The pluripotent stem cell-derived RPE cell of claim 1, wherein said iPSC medium comprises E8, Stem Flex, or mTeSR1 as a basal medium, and is supplemented with 10 µM Y27632 as an additive.

5. The pluripotent stem cell-derived RPE cell of claim 1, wherein said RPE cell is obtained through differentiation of iPSCs in an E6 medium supplemented with chemical small molecules serving as inhibitors or activators, said differentiation comprises the following stages: a neuroectodermal cell induction stage, an RPE progenitor cell induction stage, an RPE cell induction stage, and an RPE cell maturation stage.

6. The pluripotent stem cell-derived RPE cell of claim 1, wherein said neuroectodermal cell induction stage comprises: inhibiting the TGF-β/Nodal/Activin signaling pathway by adding 1-50 µM of small molecule SB431542; inhibiting the BMP signaling pathway by adding 10-100 nM of small molecule LDN193183; and inhibiting the WNT signaling pathway by adding 10-100 µM of small molecule IWR-1 or IWR-2.

7. The pluripotent stem cell-derived RPE cell of claim 1, wherein said RPE progenitor cell induction stage comprises activating the Nicotinamide metabolic signaling pathway by adding 1-50 mM of nicotinamide.

8. The pluripotent stem cell-derived RPE cell of claim 1, wherein said RPE cell induction stage comprises: activating the TGF-β/Nodal/Activin signaling pathway by adding 10-200 ng/mL of Activin A; inhibiting the FGF signaling pathway by adding 1-10 µM of small molecule SU5402; and activating the WNT signaling pathway by adding 1-10 µM of small molecule CHIR99021.

9. The pluripotent stem cell-derived RPE cell of claim 1, wherein said RPE cell maturation stage comprises: activating the Nicotinamide metabolic signaling pathway by adding 1-50 mM of Nicotinamide; and activating the WNT signaling pathway by adding 1-10 µM of small molecule CHIR99021.

10. The pluripotent stem cell-derived RPE cell of claim 1, wherein during the passaging from said RPE cell induction stage to said RPE cell maturation stage, the split ratio is 1:(3-20).

11. An RPE cell preparation, comprising said pluripotent stem cell-derived RPE cell of any one of claims 1 to 10.

12. A pharmaceutical composition for use in ophthalmology, comprising said pluripotent stem cell-derived RPE cell of any one of claims 1 to 10 as an active ingredient.

13. A reagent for evaluating the toxicity or efficacy of a test substance, comprising said pluripotent stem cell-derived RPE cell of any one of claims 1 to 10.

14. A method for evaluating the toxicity or efficacy of a test substance, comprising contacting said pluripotent stem cell-derived RPE cell of any one of claims 1 to 10 with said substance and determining the effect of said substance on said cell.

15. A kit, comprising a medium combination; wherein said medium combination comprises:
a first medium, a second medium, a third medium, and a fourth medium;
said first medium comprises a basal medium and additive A, wherein said additive A is one or more selected from SB431542, LDN193183, IWR-1, and IWR-2 in any molar ratio; wherein SB431542 is added at 1-50 µM, LDN193183 at 10-100 nM, IWR-1 at 10-100 µM, and IWR-2 at 10-100 µM;
said second medium comprises a basal medium and additive B, wherein said additive B is Nicotinamide, which is added at 1-50 mM;
said third medium comprises a basal medium and additive C, wherein additive C is one or more selected from Activin A, SU5402, and CHIR99021 in any molar ratio; wherein Activin A is added at 10-200 ng/mL, SU5402 at 1-10 µM, and CHIR99021 at 1-10 µM;
said fourth medium comprises a basal medium and additive D, wherein additive D is one or both of Nicotinamide and CHIR99021 in any molar ratio; wherein Nicotinamide is added at 1-50 mM and CHIR99021 at 1-10 µM.

16. The kit of claim 15, wherein said basal medium is E6 medium.

17. A method of obtaining RPE cells using said kit of claim 15 or 16, comprising the following steps: Step 1: culturing pluripotent stem cells in said first medium for 2 days to obtain neuroectodermal cells; Step 2: culturing said neuroectodermal cells in said second medium for 4 days to obtain RPE progenitor cells; Step 3: culturing said RPE progenitor cells in said third medium for 4 days to obtain RPE cells; Step 4: culturing said RPE cells in said fourth medium for 20 days to obtain mature RPE cells.
